# EUROPÄISCHE PATENTSCHRIFT

(11) **EP 2 585 429 B1**
(45) Veröffentlichungstag und Bekanntmachung des Hinweises auf die Patenterteilung: **09.08.2017**
(21) Anmeldenummer: 11726701.3
(22) Anmeldetag: 31.05.2011
(51) Int. Cl.: C07C 51/42, C07C 51/43, C07C 51/47, C07C 55/02, C07C 55/10, C07C 55/14, C07C 57/145, C07B 63/00, C12P 7/44, C07C 63/06, C12P 7/46

(54) **VERFAHREN ZUR ABTRENNUNG, GEWINNUNG UND REINIGUNG VON DICARBONSÄUREN**
PROCESS FOR REMOVING, ISOLATING AND PURIFYING DICARBOXYLIC ACIDS
PROCÉDÉ POUR SÉPARER, OBTENIR ET PURIFIER DES ACIDES DICARBOXYLIQUES

(30) Priorität: 25.06.2010 DE 102010025167
(43) Veröffentlichungstag der Anmeldung: 01.05.2013
(73) Patentinhaber: ThyssenKrupp Industrial Solutions AG, 45143 Essen (DE)
(72) Erfinder: TIETZ, Wolfgang, 06408 Biendorf (DE); SCHULZE, Joachim, 59494 Soest (DE)
(74) Vertreter: thyssenkrupp Intellectual Property GmbH
(86) Internationale Anmeldenummer: PCT/EP2011/002686
(87) Internationale Veröffentlichungsnummer: WO 2011/160760

(56) Entgegenhaltungen:
- EP-A1- 1 669 459
- WO-A1-2010/003534
- US-A- 5 177 008

## Beschreibung

Bernsteinsäure und ihre Verbindungen werden in großem Umfang für verschiedene Anwendungen in der Lebensmittelindustrie, der Pharmazie, der Kosmetik und der Textilindustrie genutzt. Zunehmend wird Bernsteinsäure als Ausgangsprodukt für die Herstellung von Polymeren benutzt.

Entscheidend für die industrielle Nutzung von Bernsteinsäure, die durch Fermentation kohlehydrathaltiger Substrate mittels verschiedener Mikroorganismen erzeugt wird, ist die Wirtschaftlichkeit und Effizienz der Abtrennung und Reinigung der Bernsteinsäure aus diesen wässrigen Fermentationslösungen, die neben der Bernsteinsäure oder Bernsteinsäuresalzen auch weitere organische Säuren, sonstige Nebenprodukte der Fermentation, Mikroorganismen und deren Bestandteile sowie Reste der Substrate enthalten.

Unterschieden werden können die Qualitäten der erzeugten Bernsteinsäure durch die Unterteilung in eine technische Qualität mit einem Bernsteinsäuregehalt von mindestens 97 Ma-% und einer speziell für die Verwendung zur Polymerisation geeigneten Bernsteinsäure (polymer grade) mit einem Gehalt von mindestens 99,5 Ma-%.

Eine Vielzahl von Patenten beschreibt die Gewinnung von Bernsteinsäure aus Fermentationslösungen, darunter
- extraktive Prozesse unter Verwendung von Extraktionsmitteln wie Tributylaminen, Trialkylaminen, Olefinen, verschiedenen Alkoholen und aromatischen Kohlenwasserstoffen,
- Prozesse unter Verwendung von Calciumhydroxid und Schwefelsäure, wobei als Nebenprodukt Gips anfällt,
- Prozesse unter Verwendung der Elektrodialyse,
- Thermische Methoden wie fraktionierte Destillation oder thermisch gestufte Chromatographie,
- Hochdruckextraktion unter Verwendung von CO₂,
- Membranverfahren wie beispielsweise Umkehrosmose und sonstige Filtrationsprozesse
wobei auch Kopplungen dieser Verfahren und Ergänzung durch weitere dem Stand der Technik entsprechende Schritte diskutiert werden. Derartige Verfahren werden unter anderem in den Patentschriften DE 69821951 T2 ; DE 69015233 T2 ; DE 69015019 T2 ; DE 69006555 T2 ; DE 69015019 ; DE19939630C2; DE 60028958T2 ; DE 10 2004 026152 A1, EP 1669459 A1 und US 5177008 A beschrieben.

Nachteil vieler Verfahren ist, dass zusätzliche Stoffe dem Prozess zugeführt werden, die im Zielprodukt nicht mehr enthalten sein dürfen bzw. deren Spuren im Zielprodukt zu Einschränkungen in der Qualität und der Anwendbarkeit des Produktes führen können. Auch ist die praktische Durchführung der Verfahren zum Teil mit erheblichem technischem und energetischem Aufwand verbunden.

Aufgabe der Erfindung ist es, ein Verfahren zur Abtrennung, Gewinnung und Reinigung von Dicarbonsäuren, wie Bernsteinsäure, aus Fermentationsbrühen zur Verfügung zu stellen, das eine hohe Produktreinheit gewährleistet und bekannte Nachteile anderer Verfahren vermeidet.

Erfindungsgemäß wird die Aufgabe gelöst, durch den Einsatz eines Verfahrens zur Abtrennung, Gewinnung und Reinigung von Dicarbonsäure aus Fermentationsbrühen durch Anwendung von mehreren Verarbeitungsstufen, wobei das Verfahren die folgenden Schritte umfasst,
a) eine Abtrennung der Biomasse und eventuell vorhandenen Feststoffen aus der Fermentationsbrühe in zwei aufeinanderfolgenden Stufen,
b) Abtrennung der Dicarbonsäurelösung aus der biomassefreien Fermentationsbrühe durch Simulated Moving Bed Chromatographie (SMB),
c) Feinreinigung der Dicarbonsäurelösung,
d) Mehrstufige Eindampfung und Kristallisation,
e) Separation und Trocknung der Kristalle,
wobei
(i) die Biomasseabtrennung aus der Fermentationsbrühe in Verfahrensschritt a) in einer ersten Stufe ohne Absenken des pH-Wertes durch Zugabe von Säure und ohne thermische Inaktivierung durch eine Precoat- und/oder Mikrofiltration erfolgt,
   wobei die Temperatur und pH-Wert den Werten der Fermentation entsprechen,
(ii) die Zeit zwischen Entnahme aus dem Fermentor und der Filtration nicht mehr als 2 h betragt, vorzugsweise weniger als 1 - 2 h betragt, und
(iii) die Biomassekonzentration im Filtrat nicht höher als 1 g/l ist.

Die Fermentationsbrühe, die die Dicarbonsäure in Form von Ammoniumsuccinat, wenn die Dicarbonsäure Bernsteinsäure ist, Biomasse und Bestandteile des Substrates enthält, wird kontinuierlich einer Precoat-Filtration oder einer Mikrofiltration zugeführt. Dabei entsprechen Temperatur und pH-Wert den Werten der Fermentation, da festgestellt wurde, dass durch Inaktivierung der Biomasse durch Temperaturerhöhung und Absenken des pH-Wertes durch Zugabe von Säure eine Autolyse der Biomasse beschleunigt wird und mehr Lyseprodukte in die Fermentationsbrühe abgegeben werden. Auch muss die Zeit zwischen Beendigung der Fermentation und der Abtrennung der Biomasse so kurz als möglich gehalten werden und nicht mehr als 2 h betragen, und vorzugsweise weniger als 1 - 2 h betragen sollte. Die Biomassekonzentration im Filtrat sollte 1 g/l nicht übersteigen. Durch diese Prozessführung wird die Endproduktqualität positiv beeinflusst.

Das Filtrat aus der Precoat- oder Mikrofiltration wird im zweiten Schritt einer ein- oder zweistufigen Ultrafiltration zugeführt. Hier werden restliche Biomasseanteile, unlösliche Feststoffe und höhermolekulare Verbindungen abgetrennt. Als Optimum zwischen Produktqualität und Fluxraten der Membranen wurden Membranen mit einer Tenngrenze von ≤10 kDa ermittelt. Die Temperatur der flüssigen Medien sollte wegen des Löslichkeitskoeffizienten von Ammoniumsuccinat in Wasser ≥ 30 °C betragen. Das Retentat wird zur Precoat- oder Mikrofiltration zurückgeführt oder alternativ als Ausgangsprodukt für die Erzeugung von Dicarbonsäure technischer Qualität gesammelt bzw. verwendet und das Permeat wird der weiteren Behandlung zugeführt.

Im Permeat der Ultrafiltration liegt die Dicarbonsäure in Form ihres Salzes - im Falle von Bernsteinsäure in Form von Ammoniumsuccinat - vor. Zur Überführung in die Dicarbonsäure erfolgt die Zugabe und Einmischung von konzentrierter Schwefelsäure und damit verbunden eine Absenkung des pH-Wertes der Lösung auf Werte zwischen 2,2 bis 2,4. Dabei entsteht in stöchiometrischem Verhältnis Ammoniumsulfat. Zur Vermeidung von unerwünschter Ausfällung erfolgt dieser Prozessschritt bei Temperaturen zwischen 30 °C bis 60 °C und vorzugsweise in einem Bereich zwischen 30°C bis 40°C gehalten wird. Diese vorgereinigte Lösung steht für die Abtrennung und Reinigung der Dicarbonsäure zur Verfügung.

Die Trennung des sauren Permeates der Ultrafiltration erfolgt in einer Simulated Moving Bed Chromatographie. Diese stellt eine besonders leistungsfähige Variante der High Performance Liquid Chromatographie dar, wobei durch die Aufeinanderfolge von mehreren über Ventile miteinander verbundenen Trennsäulen in einer Endlosschleife eine große Anzahl theoretischer Böden verwirklicht und die Trennschärfe der Chromatographie erheblich verbessert wird. Als stationäre Phase kommen Kationenaustauscher und Anionenaustauscher zum Einsatz. Nach Aufgabe der Lösung wird die Dicarbonsäure an die stationäre Phase gebunden und nach mehrfacher Ausspülung der nicht gewünschten Anteile der Lösung aus dem System eluiert und als Extrakt gesondert abgeführt. Als Eluent kommen entmineralisiertes Wasser und/oder Brüdenkondensat zur Verwendung. Es konnte gezeigt werden, dass im Extrakt mehr als 95 % der im Permeat der Ultrafiltration enthaltenen Dicarbonsäure gewonnen werden kann, wobei das Verhältnis zwischen Permeat der Ultrafiltration und Eluent im Bereich zwischen 1 : 1 und 1 : 2,5 variiert und acht in einer Endlosschleife geschalteten Anionenaustauscher-Säulen Verwendung fanden. Der Extrakt enthält nur noch geringe Mengen an Ammoniumsulfat, Essigsäure und Farbstoffe aus der Fermentorbrühe. Das ausgespülte Raffinat enthält maximal 1 g/l Dicarbonsäure sowie das Ammoniumsulfat, Begleitsalze aus der Fermentation wie Phosphate, Nitrate und Chloride.

Für die Herstellung einer hochreinen Dicarbonsäure (polymer grade) erfolgt optional eine Feinreinigung des Extraktes aus der Simulated Moving Bed Chromatographie, wobei die Membranen eine Trenngröße von 100 bis 400 Da aufweisen. Es konnte gezeigt werden, dass eine Nanofiltration mit einer Trenngrenze um 200 Da gute Qualitätsergebnisse ergibt. Dabei wird der Prozess so geführt, dass das Retentat der Nanofiltration nicht mehr als 10% des Gesamtdurchsatzes beträgt. Das Retentat enthält neben Dicarbonsäure noch Essigsäure sowie Farbstoffe und kann dem Ausgangsprodukt für die Erzeugung von Dicarbonsäure in technischer Qualität zugegeben werden. Das Permeat wird der weiteren Verarbeitung zu polymer grade Dicarbonsäure zugeleitet.

In Abhängigkeit der Qualität der für die Fermentation eingesetzten Rohstoffe und der Prozessführung in der Fermentation wird aufgrund noch vorhandener Reste von Farbstoffen und Begleitstoffen eine zusätzliche Feinreinigung des Permeates aus der vorgeschalteten Nanofiltration oder des Extraktes aus der SMB-Chromatographie durchgeführt. In diesem Fall wird eine Feinreinigung durch Aktivkohlefiltration und/oder Ionenaustauscher nachgeschaltet. Als Ionenaustauscherharze kommen in Abhängigkeit von der chemischen Analyse der Verunreinigungen Kationen- und/oder Anionenaustauscher in Betracht.

Die Darstellung des Produktes Dicarbonsäure - sowohl in technischer als auch in polymer grade Qualität - erfolgt durch Eindampfung der Dicarbonsäurelösung und anschließende Kristallisation. Dabei wurde festgestellt, dass die Parameter der Prozessführung erheblichen Einfluss auf die Produktqualität haben.

Für die Erzielung einer technischen Qualität mit einem Dicarbonsäuregehalt von ≥ 97 Ma-% ist es ausreichend, wenn Eindampfung und Kristallisation in einem einmaligen Durchlauf durch diese mehrstufigen Verfahrensschritte erfolgen. Die Eindampfung der Lösung erfolgt bis zu einer Konzentration von 30 bis 50 Ma.-%. Als wesentlicher Parameter für die Produktqualität wurde der während der Kristallisation anzuwendende Temperaturgradient für die Kühlung der Lösung gefunden. Die Abkühlung sollte demnach in Schritten von 3 - 8 °C/min erfolgen und bevorzugt in Schritten von 3 - 5°C/min. Die erzeugten Kristalle werden dann von der Mutterlauge durch Separation abgetrennt, mit Warmwasser von 40 °C gewaschen und die Mutterlauge vor die Eindampfung zurückgeführt. Die Kristalle werden nach der Separation getrocknet.

Für die Erzielung einer polymer grade- Qualität der Dicarbonsäure mit einem Gehalt von ≥ 99,5 Ma-% wurde gefunden, dass dabei die Temperatur im Bereich von 70 °C bis 80 °C liegen sollte und die Lösung auf eine Konzentration von 50 ± 5 Ma-% eingestellt werden muss. Als wesentlich für die Qualität der Kristalle wurde der Temperaturgradient während der Kühlung der Lösung festgestellt. Demnach erfolgt die Abkühlung in Schritten von 1 °C bis 5 °C/h. Damit werden Kristalle in polymer grade - Qualität erzeugt, die durch Separation abgetrennt und getrocknet werden. Die Mutterlauge kann zurückgeführt werden. Erforderlichenfalls kann nach der Separation eine Auflösung der Kristalle mit entmineralisiertem Wasser und/oder Brüdenkondensat erfolgen und der Schritt der Kristallisation und Separation wiederholt werden.

Vorteilhaft werden die Kristalle der Dicarbonsäure nach der Kristallisation durch Separation abgetrennt, wobei eine anfallende Mutterlauge vor die Eindampfung zurückgeführt wird und anschließend eine Trocknung der Kristalle erfolgt.

Optional werden die Retentate der Ultrafiltration und der Nanofiltration zusammengeführt und als Ausgangslösung für die Herstellung einer Dicarbonsäure in technischer Qualität (technical grade) dienen. Die getrockneten Kristalle werden für die weitere Verwendung konfektioniert.

Mit Vorteil wird das erfindungsgemäße Verfahren zur Reinigung von Dicarbonsäuren, ausgewählt aus der Gruppe Fumarsäure, Maleinsäure, Adipinsäure, Itaconsäure, und weiteren, insbesondere von Bernsteinsäure angewendet.

### Beispiel 1

Eine Ammonuimsuccinat enthaltende Fermentorbrühe wurde entsprechend der Beschreibung durch Filtration vorgereinigt. Nach Überführung des Ammoniumsalzes in die Säureform der Bernsteinsäure wurde die Lösung in einer Simulated Moving Bed Chromatographie in 5,7 I Extrakt und 6,6 I Raffinat aufgetrennt. Dabei wurden insgesamt 8 Trennsäulen mit einem stark sauren Kationenaustauscher zu einer Endlosschleife verschaltet. Bei einem Permeat-/ Eluentverhältnis von 2,4 betrug der Koeffizient der Bernsteinsäuregewinnung 99,9 %. Die Sulfatkonzentration im Extrakt betrug 238 mg/l und im Raffinat 35.709 mg/l, womit eine Sulfatelimination von 99,4% erzielt wurde.

### Beispiel 2

Eine Ammonuimsuccinat enthaltende Fermentorbrühe wurde entsprechend der Beschreibung durch Filtration vorgereinigt. Nach Überführung des Ammoniumsalzes in die Säureform der Bernsteinsäure wurde die Lösung in einer Simulated Moving Bed Chromatographie in 5,3 I Extrakt und 6,1 I Raffinat aufgetrennt. Dabei wurden insgesamt 8 Trennsäulen mit einem stark sauren Kationenaustauscher zu einer Endlosschleife verschaltet. Bei einem Permeat-/ Eluentverhältnis von 2,2 betrug der Koeffizient der Bernsteinsäuregewinnung 99,8 %. Es wurde eine Sulfatelimination von 97,9% erzielt

### Beispiel 3

Ein die Bernsteinsäure enthaltender Extrakt aus der Simulated Moving Bed Chromatographie wurde einer Feinreinigung durch Nanofiltration mit einer Trenngrenze von 200 Da unterzogen. Im Extrakt wurden Gehalte von 44,8 g/l Bernsteinsäure und 698 mg/l Sulfate analysiert. Der filtrierte Extrakt wurde kristallisiert und analysiert. Die Kristalle hatten einen Bernsteinsäuregehalt von 1.031 g/l und einen Gehalt an restlichen Sulfaten von 21,9 mg/l und Chloriden von 13,8 mg/l. Die Farbe der Kristalle war "weiß".

### Beispiel 4

Ein die Bernsteinsäure enthaltender Extrakt aus der Simulated Moving Bed Chromatographie mit einem Gehalt von 44,77 g/l Bernsteinsäure und 699 mg/l Sulfaten wurde einer Feinreinigung durch Nanofiltration mit einer Trenngrenze von 200 Da und folgender Aktivkohlefiltration unterzogen. Die nach der Feinreinigung erzeugten Kristalle wiesen einen Gehalt an Bernsteinsäure von 1.065 g/l und restlichen Sulfaten von 35,3 mg/l sowie von 9,5 mg/l Chloriden auf. Die Farbe der Kristalle war "reinweiß".

### Beispiel 5

Ein die Bernsteinsäure enthaltender Extrakt aus der Simulated Moving Bed Chromatographie wurde einer Feinreinigung mittels Ionenaustausch unterzogen. Der Extrakt hatte einen Gehalt von 44,8 g/l Bernsteinsäure, 699 mg/l Sulfate und 1.88 mg/l Chloride. Die aus der feingereinigten Lösung erzeugten Kristalle wiesen einen Gehalt an Bernsteinsäure von 967 g/l Bernsteinsäure, 37,6 mg/l Sulfate und 0,92 mg/l Chloride auf. Die Farbe der Kristalle war "weiß.

## Patentansprüche

1. Verfahren zur Abtrennung, Gewinnung und Reinigung von Dicarbonsäuren aus Fermentationsbrühen, wobei das Verfahren die folgenden Schritte umfasst:
(a) eine Abtrennung der Biomasse und eventuell vorhandenen Feststoffen aus der Fermentationsbrühe in zwei aufeinander folgenden Stufen,
(b) Abtrennung der Dicarbonsäurelösung aus der biomassefreien Fermentationsbrühe durch Simulated Moving Bed Chromatographie (SMB),
(c) Feinreinigung der Dicarbonsäurelösung,
(d) Mehrstufige Eindampfung und Kristallisation, und
(e) Separation und Trocknung der Kristalle,
wobei
(i) die Biomasseabtrennung aus der Fermentationsbrühe in Verfahrensschritt a) in einer ersten Stufe ohne Absenken des pH-Wertes durch Zugabe von Säure und ohne thermische Inaktivierung durch eine Precoat- und/oder Mikrofiltration erfolgt,
wobei die Temperatur und pH-Wert den Werten der Fermentation entsprechen,
(ii) die Zeit zwischen Entnahme aus dem Fermentor und der Filtration nicht mehr als 2 h betragt, vorzugsweise weniger als 1 - 2 h betragt, und
(iii) die Biomassekonzentration im Filtrat nicht höher als 1 g/l ist.

2. Verfahren nach Anspruch 1, **dadurch gekennzeichnet,**
**dass** in Verfahrensschritt a) die zweite Stufe der Biomasse- und Feststoffabtrennung durch ein oder zweistufige Ultrafiltration mit Membranen der Trenngrenze von ≤ 10 kDa erfolgt, wobei ein entstehendes Retentat zur Precoat- oder Mikrofiltration der ersten Stufe des Verfahrensschrittes a) zurückgeführt wird und das entstehende Permeat der weiteren Behandlung zugeführt wird.

3. Verfahren nach einem der Ansprüche 1 und/oder 2, **dadurch gekennzeichnet,**
**dass** das Permeat der Ultrafiltration aus der zweiten Stufe des Verfahrensschrittes a) mit konzentrierter Schwefelsäure auf einen pH-Wert von 2,2 bis 2,4 angesäuert wird, somit das in der gereinigten Fermentationslösung enthaltene Salz der Dicarbonsäure in Dicarbonsäure überführt wird und im stöchiometrischen Verhältnis Salz entsteht, wobei die Temperatur des angesäuerten Permeates der Ultrafiltration in einem Bereich zwischen 30 °C bis 60 °C, und vorzugsweise in einem Bereich zwischen 30 bis 40 °C gehalten wird.

4. Verfahren nach einem der Ansprüche 1 bis 3, **dadurch gekennzeichnet, dass** das Permeat der Ultrafiltration in einer Simulated Moving Bed Chromatographie (SMB) in Verfahrensschritt b) in einen die Hauptmenge der Dicarbonsäure enthaltenden Extrakt und einen die Hauptmenge Ammoniumsulfat sowie geringe Mengen an Begleitsalzen wie Phosphate, Nitrate und Chloride enthaltendes Raffinat getrennt wird, wobei
(i) die Zugabe des Permeates der Ultrafiltration und eines Eluents kontinuierlich in einem Verhältnis Permeat: Eluent von 1: 1,5 bis 1: 2,5 erfolgt,
(ii) die Dicarbonsäure an eine stationäre Phase der SMB bindet, wobei diese aus einem Kationenaustauscher und/oder einem Anionenaustauscher aufgebaut ist,
(iii) der die Dicarbonsäure enthaltende Extrakt, aufweisend einen Gehalt an Dicarbonsäure ≤ 1 g/l, und das Raffinat getrennt voneinander gesammelt werden, und
(iv) der Wirkungsgrad der Gewinnung von Dicarbonsäure aus dem Permeat der Ultrafiltration ≥ 95% beträgt.

5. Verfahren nach Anspruch 4, **dadurch gekennzeichnet, dass** der Extrakt aus der SMB-Chromatographie in Verfahrensschritt c) einer Nanofiltration unterzogen wird, wobei die Membranen eine Trenngrenze von 100 bis 400 Da, vorzugsweise 200 Da, besitzen.

6. Verfahren nach einem der Ansprüche 1 bis 5, **dadurch gekennzeichnet, dass** in Verfahrensschritt c) eine Feinreinigung durch Aktivkohlefiltration und/oder Kationenaustauscher und/oder Anionenaustauscher vorgesehen ist und eine gereinigte Dicarbonsäure hergestellt wird.

7. Verfahren nach einem der Ansprüche 1 bis 6, **dadurch gekennzeichnet, dass** die Dicarbonsäure in Verfahrensschritt d) durch eine mehrstufige Eindampfung der gereinigten Dicarbonsäurelösung aufkonzentriert und nachfolgend durch Kristallisation in Form von Kristallen gewonnen wird, wobei
(i) für die Erzielung einer technischen Qualität der Dicarbonsäure mit einem Gehalt von ≥ 97 Massen-% Dicarbonsäure ein einzelner Durchlauf durch die mehrstufige Eindampfung und Kristallisation erfolgt und die Abkühlung der konzentrierten Dicarbonsäure in einem Kristallisator in Schritten von 3 - 8 °C/min., vorzugsweise 3 - 5 °C/min., erfolgt, und
(ii) für die Erzielung einer hochreinen Dicarbonsäure mit einem Gehalt von ≤ 99,5 Massen-% (polymer-grade) die Lösung auf eine Temperatur von 70 - 80 °C und eine Konzentration von 50 ± 5 Massen-% eingestellt wird, und nachfolgend eine Kristallisation durchgeführt wird, wobei die Abkühlung des Konzentrates in Schritten von 1- 5 °C/h erfolgt und dieser Schritt erforderlichenfalls nach Separation der Kristalle und Auflösung in entmineralisiertem Wasser und/oder Brüdenkondensat wiederholt wird.

8. Verfahren nach einem der Ansprüche 1 bis 7, **dadurch gekennzeichnet, dass** die Kristalle der Dicarbonsäure nach der Kristallisation in Verfahrensschritt e) durch Separation abgetrennt werden, wobei eine anfallende Mutterlauge vor die Eindampfung in Verfahrensschritt d) zurückgeführt wird und anschließend eine Trocknung der Kristalle erfolgt.

9. Verfahren nach einem der Ansprüche 1 bis 8, **dadurch gekennzeichnet, dass** die Retentate der Ultrafiltration aus der zweiten Stufe des Verfahrensschritts a) und der Nanofiltration aus Verfahrensschritt c) zusammengeführt werden und als Ausgangslösung für die Herstellung einer Dicarbonsäure in technischer Qualität (technical grade) dienen und die Prozessstufen weiter durchlaufen.

10. Verfahren nach einem der Ansprüche 1 bis 9, **dadurch gekennzeichnet, dass** das Verfahren zur Reinigung von Dicarbonsäuren, ausgewählt aus der Gruppe Fumarsäure, Maleinsäure, Adipinsäure, Itaconsäure, und weiteren, insbesondere von Bernsteinsäure angewendet wird.

## Claims

1. Process for separating, recovering and purifying dicarboxylic acids from fermentation broths, said process comprising the following steps:
a) Separation of the biomass and any solids that are present from the fermentation broth in two consecutive steps,
b) Separation of the dicarboxylic acid solution from the biomass-free fermentation broth by means of simulated moving-bed chromatography (SMB),
c) Fine purification of the dicarboxylic acid solution,
d) Multi-stage evaporation and crystallisation, and
e) Separation and drying of the crystals,
wherein
(i) the biomass is separated out from the fermentation broth in process step a) in a first stage without lowering the pH value by adding acid and without thermal inactivation by means of a precoat and/or microfiltration unit,
the temperature and pH value corresponding to the fermentation values,
(ii) the time between removal from the fermenter and the filtration does not exceed 2 h and preferably is less than 1 - 2 h, and
(iii) the biomass concentration in the filtrate does not exceed 1 g/l.

2. Process according to claim 1, **characterised in that** in process step a) the second stage in separating out the biomass and solids is carried out by means of a single- or dual-stage ultrafiltration unit employing membranes with a cut-off of ≤ 10 kDa, a retentate produced being recycled to the precoat or microfiltration unit of the first stage of process step a) and the permeate produced being sent for further treatment.

3. Process according to one of claims 1 and/or 2, **characterised in that** the ultrafiltration permeate from the second stage of process step a) is acidified to a pH value of 2.2 to 2.4 using concentrated sulphuric acid, thus converting the dicarboxylic acid salt contained in the purified fermentation solution to dicarboxylic acid and producing salt in a stoichiometric ratio, the temperature of the acidified ultrafiltration permeate being kept in a range between 30°C and 60°C, and preferably in a range between 30 and 40°C.

4. Process according to one of claims 1 to 3, **characterised in that** in process step b) the ultrafiltration permeate is separated in a simulated moving-bed chromatography (SMB) unit into an extract containing the bulk of the dicarboxylic acid and a raffinate containing the bulk of the ammonium sulphate as well as small amounts of companion salts, such as phosphates, nitrates and chlorides,
(i) the ultrafiltration permeate and an eluent being added continuously in a permeate : eluent ratio of 1 : 1.5 to 1 : 2.5,
(ii) the dicarboxylic acid binding to a stationary phase of the SMB that comprises a cation exchanger and/or an anion exchanger,
(iii) the dicarboxylic acid-containing extract with a dicarboxylic acid content of ≤ 1 g/l and the raffinate being collected separately from each other, and
(iv) the dicarboxylic acid being recovered from the ultrafiltration permeate with an efficiency ≥ 95%.

5. Process according to claim 4, **characterised in that** the extract from the SMB chromatography unit is subjected to nanofiltration in process step c), the membranes having a cut-off of 100 to 400 Da and preferably 200 Da.

6. Process according to one of claims 1 to 5, **characterised in that** process step c) envisages fine purification by means of activated carbon filtration and/or cation exchangers and/or anion exchangers, with a purified dicarboxylic acid being produced.

7. Process according to one of claims 1 to 6, **characterised in that** in process step d) the dicarboxylic acid is concentrated via multi-stage evaporation of the purified dicarboxylic acid solution before being recovered in the form of crystals via crystallisation, with
(i) a single pass through the multi-stage evaporation and crystallisation, and cooling of the concentrated dicarboxylic acid in a crystalliser in steps of 3 - 8°C/min., and preferably 3-5°C/min., to obtain technical-grade dicarboxylic acid with a dicarboxylic acid content of ≥ 97 wt.%
(ii) the solution being adjusted to a temperature of 70 - 80°C and a concentration of 50±5 wt.% with subsequent crystallisation, with cooling of the concentrate in steps of 1 - 5°C/h and if necessary repetition of this step after separation of the crystals and dissolution in demineralised water and/or vapour condensate to obtain polymer-grade dicarboxylic acid with a content of ≤ 99.5 wt.%.

8. Process according to one of claims 1 to 7, **characterised in that** after crystallisation the dicarboxylic acid crystals are removed by means of separation in process step e) with the accumulating mother liquor being recycled to upstream of the evaporation in process step d) and the crystals subsequently being dried.

9. Process according to one of claims 1 to 8, **characterised in that** the ultrafiltration retentates from the second stage of process step a) and the nanofiltration retentates from process step c) are combined and used as a feedstock solution for the production of technical-grade dicarboxylic acid, continuing to pass through the process stages.

10. Process according to one of claims 1 to 9, **characterised in that** the process is used for the purification of dicarboxylic acids selected from the group fumaric acid, maleic acid, adipic acid, itaconic acid and others, in particular of succinic acid.

## Revendications

1. Procédé pour séparer, obtenir et purifier des acides dicarboxyliques à partir de bouillons de fermentation, le procédé comprenant les étapes suivantes :
(a) une séparation de la biomasse et de solides éventuellement présents du bouillon de fermentation en deux étapes successives,
(b) la séparation de la solution d'acides dicarboxyliques du bouillon de fermentation sans biomasse par chromatographie SMB (Simulated Moving Bed),
(c) la purification fine de la solution d'acides dicarboxyliques,
(d) l'évaporation en plusieurs étapes et la cristallisation, et
(e) la séparation et le séchage des cristaux,
(i) la séparation de la biomasse du bouillon de fermentation à l'étape de procédé a) ayant lieu lors d'une première étape sans abaissement du pH par ajout d'un acide et sans inactivation thermique par une filtration à précouches et/ou une microfiltration,
la température et le pH correspondant aux valeurs de la fermentation,
(ii) le temps entre le soutirage du fermentateur et la filtration n'étant pas supérieur à 2 h, de préférence inférieur à 1 à 2 h, et
(iii) la concentration de la biomasse dans le filtrat n'étant pas supérieure à 1 g/l.

2. Procédé selon la revendication 1, **caractérisé en ce qu'**à l'étape de procédé a), la deuxième étape de la séparation de la biomasse et des solides a lieu par ultrafiltration à une ou deux étapes avec des membranes ayant une limite de séparation ≤ 10 kDa, un rétentat formé étant recyclé dans la filtration à précouches ou la microfiltration de la première étape de l'étape de procédé a) et le perméat formé étant introduit dans le traitement ultérieur.

3. Procédé selon l'une quelconque des revendications 1 et/ou 2, **caractérisé en ce que** le perméat de l'ultrafiltration de la deuxième étape de l'étape de procédé a) est acidifié avec de l'acide sulfurique concentré à un pH de 2,2 à 2,4, le sel de l'acide dicarboxylique contenu dans la solution de fermentation purifiée étant ainsi transformé en acide dicarboxylique et un sel se formant en un rapport stoechiométrique, la température du perméat de l'ultrafiltration acidifié étant maintenue dans une plage comprise entre 30 °C et 60 °C, et de préférence dans une plage comprise entre 30 et 40 °C.

4. Procédé selon l'une quelconque des revendications 1 à 3, **caractérisé en ce que** le perméat de l'ultrafiltration est séparé dans une chromatographie SMB (Simulated Moving Bed) à l'étape de procédé b) en un extrait contenant la quantité principale de l'acide dicarboxylique et un raffinat contenant la quantité principale de sulfate d'ammonium, ainsi que de petites quantités de sels accompagnateurs, tels que des phosphates, des nitrates et des chlorures,
(i) l'ajout du perméat de l'ultrafiltration et d'un éluant ayant lieu en continu en un rapport perméat:éluant de 1:1,5 à 1:2,5,
(ii) l'acide dicarboxylique se liant à une phase stationnaire de la chromatographie SMB, celle-ci étant formée par un échangeur de cations et/ou un échangeur d'anions,
(iii) l'extrait contenant l'acide dicarboxylique, présentant une teneur en acide dicarboxylique ≤ 1 g/l, et le raffinat étant recueillis séparément l'un de l'autre, et
(iv) le degré d'efficacité de l'obtention de l'acide dicarboxylique à partir du perméat de l'ultrafiltration étant ≥ 95 %.

5. Procédé selon la revendication 4, **caractérisé en ce que** l'extrait de la chromatographie SMB est soumis à l'étape de procédé c) à une nanofiltration, les membranes présentant une limite de séparation de 100 à 400 Da, de préférence de 200 Da.

6. Procédé selon l'une quelconque des revendications 1 à 5, **caractérisé en ce qu'**à l'étape de procédé c), une purification fine est prévue par filtration sur charbon actif et/ou échangeurs de cations et/ou échangeurs d'anions, et un acide dicarboxylique purifié est fabriqué.

7. Procédé selon l'une quelconque des revendications 1 à 6, **caractérisé en ce que** l'acide dicarboxylique est concentré à l'étape de procédé d) par une évaporation à plusieurs étapes de la solution d'acide dicarboxylique purifiée, puis obtenu par cristallisation sous la forme de cristaux,
(i) pour l'obtention d'une qualité technique de l'acide dicarboxylique ayant une teneur ≥ 97 % en masse en acide dicarboxylique, un passage unique dans l'évaporation à plusieurs étapes et la cristallisation ayant lieu et le refroidissement de l'acide dicarboxylique concentré ayant lieu dans un cristallisateur en étapes de 3 à 8 °C/minute, de préférence de 3 à 5 °C/minute, et
(ii) pour l'obtention d'un acide dicarboxylique hautement pur ayant une teneur ≤ 99,5 % en masse (qualité polymère), la solution étant ajustée à une température de 70 à 80 °C et à une concentration de 50 ± 5 % en masse, puis une cristallisation étant réalisée, le refroidissement du concentré ayant lieu en étapes de 1 à 5 °C/h et cette étape étant si nécessaire répétée après séparation des cristaux et dissolution dans de l'eau déminéralisée et/ou un condensat de vapeur.

8. Procédé selon l'une quelconque des revendications 1 à 7, **caractérisé en ce que** les cristaux de l'acide dicarboxylique sont séparés après la cristallisation à l'étape de procédé e) par séparation, une liqueur mère formée étant recyclée avant l'évaporation à l'étape de procédé d), puis un séchage des cristaux ayant lieu.

9. Procédé selon l'une quelconque des revendications 1 à 8, **caractérisé en ce que** les rétentats de l'ultrafiltration de la deuxième étape de l'étape de procédé a) et de la nanofiltration de l'étape de procédé c) sont rassemblés et servent de solution de départ pour la fabrication d'un acide dicarboxylique de qualité technique (technical grade) et traversent encore les étapes de procédé.

10. Procédé selon l'une quelconque des revendications 1 à 9, **caractérisé en ce que** le procédé est appliqué à la purification d'acides dicarboxyliques choisis dans le groupe constitué par l'acide fumarique, l'acide maléique, l'acide adipique, l'acide itaconique et d'autres, notamment d'acide succinique.
